(19) Europäisches Patentamt
European Patent Office
Office européen des brevets

(11) Publication number: **0 008 446**
**B1**

(12) # EUROPEAN PATENT SPECIFICATION

(45) Date of publication of patent specification: **22.09.82**

(51) Int. Cl.³: **C 07 D 209/86**
//C07C59/353, A61K31/40

(21) Application number: **79103010.9**

(22) Date of filing: **17.08.79**

(54) **Process for the preparation of carbazoles and novel carbazole derivatives utilized therein.**

(30) Priority: **21.08.78 US 935197**

(43) Date of publication of application:
**05.03.80 Bulletin 80/5**

(45) Publication of the grant of the patent:
**22.09.82 Bulletin 82/38**

(84) Designated Contracting States:
**AT BE CH DE FR GB IT LU NL SE**

(56) References cited:
**US - A - 3 896 145**

(73) Proprietor: **F. HOFFMANN-LA ROCHE & CO. Aktiengesellschaft**
**CH-4002 Basel (CH)**

(72) Inventor: **Gurien, Harvey**
**15 Johnson Road**
**West Orange, N.J. (US)**
Inventor: **Teitel, Sidney**
**35 Allwood Place**
**Clifton, N.J. (US)**

(74) Representative: **Lederer, Franz, Dr. et al,**
**Patent Attorneys Dr. Franz Lederer Reiner F. Meyer 22 Lucile-Grahn-Strasse**
**D-8000 Munich 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

Process for the preparation of carbazoles and novel carbazole derivatives utilized therein

The invention relates to a process for the preparation of a compound of the formula

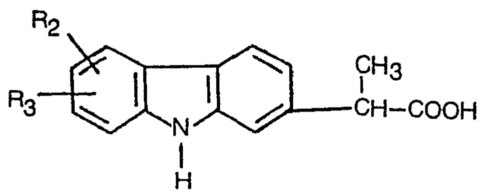

I

wherein $R_2$ is hydrogen, halogen, trifluoromethyl, hydroxy, lower alkyl, hydroxy-lower alkyl, lower alkylthio, amino, mono-lower alkylamino or di-lower alkylamino; and $R_3$ is halogen, trifuloromethyl, lower alkyl, hydroxy-lower alkyl, lower alkoxy, lower alkylthio, hydroxy, amino, mono-lower alkylamino or di-lower alkylamino or $R_2$, take together with an adjacent $R_3$, is lower alkylenedioxy, utilizing as starting materials a cyclohexanone derivative and a known phenylhydrazine of the formula III given below, which process comprises

a) reacting an $\alpha$-methyl-3-oxocyclohexane malonic acid di-lower alkyl ester of the formula

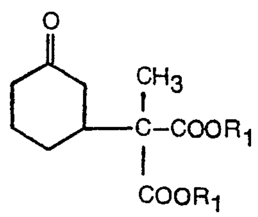

II

wherein $R_1$ is lower alkyl, with a phenylhydrazine of the formula

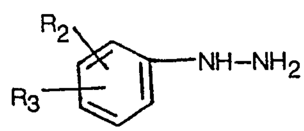

III

wherein $R_2$ and $R_3$ are as previously described; to yield a compound of the formula

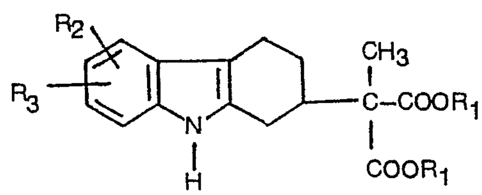

IV

wherein $R_1$, $R_2$ and $R_3$ are as previously described;

b) treating the compound of formula IV with an oxidizing agent to yield a compound of the formula

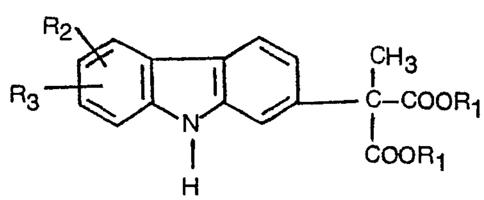

V

wherein $R_1$, $R_2$ and $R_3$ are as previously described,

c) hydrolyzing and decarboxylating the compound of formula V.

The term "lower alkyl" denotes a straight or branched chain hydrocarbon group containing 1—7 carbon atoms, e.g. methyl, ethyl, propyl, isopropyl, butyl, isobutyl or heptyl. The terms "lower alkoxy" and "lower alkylthio" denote alkyloxy and alkyl thioether groups in which the alkyl group is as described above. The term "halogen" denotes bromine, chlorine, fluorine or iodine; bromine and chlorine are preferred. The term "lower alkylene" denotes a straight or branched chain alkylene of 1—7 carbon atoms, e.g. methylene, ethylene, propylene, butylene or methylmethylene. The term "lower alkylenedioxy" preferably denotes methylenedioxy.

Exemplary of mono-lower alkylamino and di-lower alkylamino are methylamino, ethylamino, dimethylamino and diethylamino. Exemplary of amino-lower alkoxy are aminomethoxy and aminoethoxy.

The compounds of formula I are useful as anti-inflammatory, analgesic and anti-rheumatic agents. A process for the preparation of such compounds is described in the US patent 3896145. The first step of this known process comprises reacting a phenylhydrazine of the above formula III with $\alpha$-methyl-3-oxocyclohexane acetic acid and not, as in the process of the present invention, with an $\alpha$-methyl-3-oxocyclohexane malonic acid di-lower alkyl ester of the above formula II.

Step a) of the above process is carried out in the presence of an inert organic solvent, e.g. an alkanol, such as methanol, ethanol or propanol, conveniently at room temperature or above, e.g. at a temperature in the range of 25 to about 100°C. The compound of formula IV can be recovered, if desired, utilizing conventional methods. However, it is also possible to utilize the reaction product in situ in the next step of the process of the invention.

Exemplary of the compounds of formula IV are:
dimethyl-(6-chloro-1,2,3,4-tetrahydro-2-carbazolyl)-methyl malonate;
diethyl-(6-chloro-1,2,3,4-tetrahydro-2-carbazolyl)-methyl malonate;
dipropyl-(6-chloro-1,2,3,4-tetrahydro-2-carbazolyl)-methyl malonate;
dibutyl-(6-chloro-1,2,3,4-tetrahydro-2-carbazolyl)-methyl malonate;
diethyl-(6-methyl-1,2,3,4-tetrahydro-2-carbazolyl)-methyl malonate;
diethyl-(6-trifluoromethyl-1,2,3,4-tetrahydro-2-carbazolyl)-methyl malonate;
diethyl-(6-hydroxy-1,2,3,4-tetrahydro-2-carbazolyl)-methyl malonate;
diethyl-(6-ethyl-1,2,3,4-tetrahydro-2-carbazolyl)-methyl malonate;
diethyl-(6-propyl-1,2,3,4-tetrahydro-2-carbazolyl)-methyl malonate;
diethyl-(6-hydroxymethyl-1,2,3,4-tetrahydro-2-carbazolyl)-methyl malonate;
diethyl-(6-methylthio-1,2,3,4-tetrahydro-2-carbazolyl)-methyl malonate;
diethyl-(6-amino-1,2,3,4-tetrahydro-2-carbazolyl)-methyl malonate;
diethyl-(6-methylamino-1,2,3,4-tetrahydro-2-carbazolyl)-methyl malonate;
diethyl-(6-dimethylamino-1,2,3,4-tetrahydro-2-carbazolyl)-methyl malonate;
diethyl-(5-chloro-1,2,3,4-tetrahydro-2-carbazolyl)-methyl malonate;
diethyl-(7-chloro-1,2,3,4-tetrahydro-2-carbazolyl)-methyl malonate; and
diethyl-(8-chloro-1,2,3,4-tetrahydro-2-carbazolyl)-methyl malonate.

Exemplary of the compounds of formula II are the dimethyl, diethyl, dipropyl, dibutyl and dipentyl esters of $\alpha$-methyl-3-oxocyclohexane malonic acid.

An example of the substituted phenylhydrazines of formula III is p-chlorophenylhydrazine.

Step b) of the process is carried out in a hydrocarbon solvent such as benzene, toluene or xylene. Exemplary of the oxidizing agents are paraquinones such as paraquinone, chloranil (tetramethyl-p-quinone), dichloroparaquinone and dicyanoparaquinone. The reaction can be carried out at room temperature to the reflux temperature of the reaction mixture, preferably at the reflux temperature.

The compound of formula V can be recovered utilizing conventional methods, e.g. crystallization. Exemplary of compounds of formula V are the 2-carbazolyl-malonates corresponding to the above listed 1,2,3,4-tetrahydro-2-carbazolyl-malonates.

Step c) of the process can be carried out, utilizing e.g. glacial acetic acid in the presence of a hydrohalic acid, such as hydrochloric acid. The resulting product of formula I is recovered utilizing conventional methods.

Example 1

To a solution of 2.5 g of sodium in 325 ml of ethanol, 200 g of diethyl methyl malonate is added in 5 minutes. After stirring for 1 hour, a solution of 100 g of 2-cyclohexen-1-one in 130 ml of ethanol is added over a period of 1 hour. Stirring is continued overnight at room temperature, after which 20 ml of acetic acid is added and the mixture is evaporated at reduced pressure. The residue is dissolved in 1.31 l of ether and washed with three 230 ml portions of water. The ether solution is filtered and dried. The ether is removed at reduced pressure and the residual oil distilled. $\alpha$-Methyl-3-oxocyclohexane malonic acid diethyl ester distills at 129—130°/0.2 mm; 211.5 g, 75.4 o/o of theory.

Example 2

A suspension of 100 g of $\alpha$-methyl-3-oxocyclohexane malonic acid diethyl ester and 66.3 g of p-chlorophenylhydrazine hydrochloride in 300 ml of ethanol is stirred under nitrogen atmosphere at room temperature for 1.5 hours, then refluxed for 1.5 hours. The reaction mixture is cooled in an ice bath and filtered. The residue is washed with the mother liquor, the presscake sucked dry, washed with three 50 ml portions of ethanol, then with 50 ml of 1:1 hexane-ethanol and dried at 40—50° at reduced pressure. The resultant solid is stirred 15 minutes under nitrogen atmosphere with 500 ml of cold water, filtered, washed with three 100 ml portions of cold water and dried at 40° at reduced pressure to give 78.8 g of diethyl-(6-chloro-1,2,3,4-tetrahydro-2-carbazolyl)-methyl malonate, mp 129—130°, 56.5% o/o of theory.

Example 3

A mixture of are placed 161.2 g of diethyl-(6-chloro-1,2,3,4-tetrahydro-2-carbazolyl)-methyl malonate, 251.9 g of chloranil and 1.65 l of xylene is refluxed 6 hours under nitrogen atmosphere and

allowed to cool overnight. The supernatant liquid is decanted and filtered, the residue triturated with three 650 ml portions of warm benzene and the supernatants are decanted and filtered. 2 l of ether is added to the combined filtrates and the mixture extracted with four 650 ml portions of 2N sodium hydroxide. The organic phase is washed with water until the washings are neutral, dried, evaporated at reduced pressure and the residue is dried. The resulting 156.3 g of solid is dissolved in 300 ml of boiling carbon tetrachloride, treated with 3.0 g of charcoal, filtered, diluted with 600 ml of hexane, heated to the boil, seeded immediately upon cessation of heating with crystals of diethyl-(6-chloro-2-carbazolyl)-methyl malonate, allowed to cool overnight while stirring under a nitrogen atmosphere and then cooled in an ice bath. The crystalline material is filtered and washed with three 100 ml portions of 2:1 hexane-carbon tetrachloride. The solid, dried at reduced pressure, yields 119.4 g of diethyl-(6-chloro-2-carbazolyl)-methyl malonate, mp 134—135°, 75.2 o/o of theory.

Example 4

A mixture of 247 g of diethyl-(6-chloro-2-carbazolyl)-methyl malonate, 1.9 l of glacial acetic acid and 1.9 l of 6N hydrochloric acid is stirred under nitrogen atmosphere and refluxed overnight and the resulting solution allowed to cool to room temperature. The solid formed is filtered, washed with three 200 ml portions of 1:1 acetic acid-water and four 300 ml portions of water and dried. The crude 6-chloro-$\alpha$-methylcarbazole-2-acetic acid (approximately 19[illegible] is dissolved in 1.2 l of cold 1N potassium hydroxide and the solution is extracted with four [illegible] ml portions of ether, and then while cooling in an ice bath under nitrogen, is acidified by the addition of 100 ml of concentrated hydrochloric acid. Stirring is continued for 15 minutes, the precipitated solid is filtered, washed with three 100 ml portions of water and dried to give 167.7 g. Final purification is achieved by crystallization from 4.7 l of boiling 1,2-dichloroethane with 8.0 g of charcoal. The solution is allowed to cool overnight. The crystals are filtered, washed with two 200 ml portions of cold dichloroethane and dried. The yield of 6-chloro-$\alpha$-methylcarbazole-2-acetic acid is 103.8 g, mp 198.5—201°, 57.3 o/o of theory.

**Claims**

1. A process for the preparation of a compound of the formula

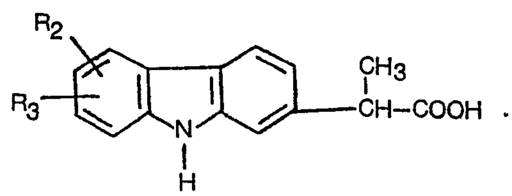

I

wherein $R_2$ is hydrogen, halogen, trifluoromethyl, hydroxy, $C_{1-7}$ alkyl, hydroxy-$C_{1-7}$ alkyl, $C_{1-7}$ alkylthio, amino, mono-$C_{1-7}$ alkylamino or di-$C_{1-7}$ alkylamino; and $R_3$ is halogen, trifluoromethyl, $C_{1-7}$ alkyl, hydroxy-$C_{1-7}$ alkyl, $C_{1-7}$ alkoxy, $C_{1-7}$ alkylthio, hydroxy, amino, mono-$C_{1-7}$ alkylamino or di-$C_{1-7}$ alkylamino, or $R_2$, taken together with an adjacent $R_3$, is $C_{1-7}$ alkylenedioxy, utilizing as starting materials a cyclohexanone derivative and a known phenylhydrazine of the formula III given below, which process comprises

a) reacting an $\alpha$-methyl-3-oxocyclohexane malonic acid di-lower alkyl ester of the formula

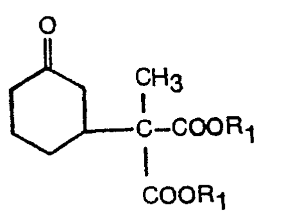

II

wherein $R_1$ is lower alkyl, with a phenylhydrazine of the formula

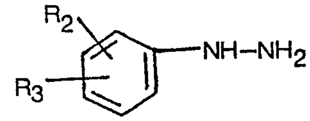

III

wherein $R_2$ and $R_3$ are as previously described, to yield a compound of the formula

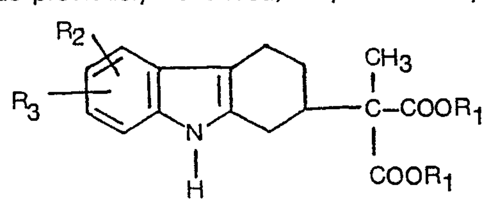

IV

wherein $R_1$, $R_2$ and $R_3$ are as previously described,

b) treating the compound of formula IV with an oxidizing agent to yield a compound of the formula

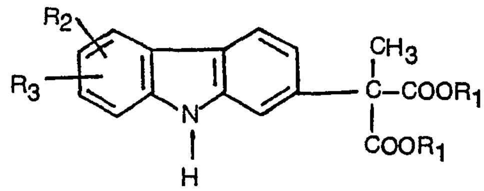

V

wherein $R_1$, $R_2$ and $R_3$ are as previously described,

c) hydrolyzing and decarboxylating the compound of formula V.

2. A process according to claim 1 in which p-chlorophenylhydrazine is utilized as starting material of formula III.

3. A compound of the formula

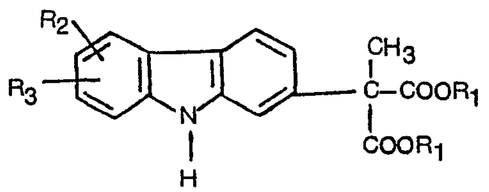

V

wherein $R_1$ is $C_{1-7}$ alkyl, $R_2$ is hydrogen, halogen, trifluoromethyl, hydroxy, $C_{1-7}$ alkyl, hydroxy-$C_{1-7}$ alkyl, $C_{1-7}$ alkylthio, amino, mono-$C_{1-7}$ alkylamino or di-$C_{1-7}$ alkylamino; and $R_3$ is halogen, trifluoromethyl, $C_{1-7}$ alkyl, hydroxy-$C_{1-7}$ alkyl, $C_{1-7}$ alkoxy, $C_{1-7}$ alkylthio, hydroxy, amino, mono-$C_{1-7}$ alkylamino or di-$C_{1-7}$ alkylamino, or $R_2$, taken together with an adjacent $R_3$, is $C_{1-7}$ alkylenedioxy.

4. Diethyl-(6-chloro-2-carbazolyl)-methyl malonate.

**Patentansprüche**

1. Ein Verfahren zur Herstellung einer Verbindung der Formel

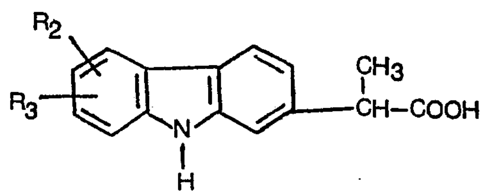

I

worin $R_2$ Wasserstoff, Halogen, Trifluormethyl, Hydroxy, $C_{1-7}$-Alkyl, Hydroxy-$C_{1-7}$-alkyl, $C_{1-7}$-Alkylthio, Amino, Mono-$C_{1-7}$-alkylamino oder Di-$C_{1-7}$-alkylamino; und $R_3$ Halogen, Trifluormethyl, $C_{1-7}$-Alkyl, Hydroxy-$C_{1-7}$-alkyl, $C_{1-7}$-Alkoxy, $C_{1-7}$-Alkylthio, Hydroxy, Amino, Mono-$C_{1-7}$-alkylamino oder Di-$C_{1-7}$-alkylamino oder $R_2$ zusammen mit einem benachbarten $R_3$ $C_{1-7}$-Alkylendioxy ist, unter Verwendung eines Cyclohexanderivates und eines bekannten Phenylhydrazins der weiter unten angegebenen Formel III, welches Verfahren dadurch gekennzeichnet ist, dass man

a) ein $\alpha$-Methyl-3-oxocyclohexan-malonsäure-di-niederalkylester der Formel

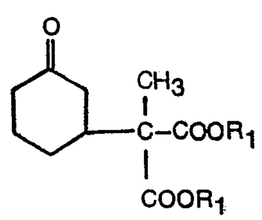

II

worin $R_1$ niederes Alkyl ist, mit einem Phenylhydrazin der Formel

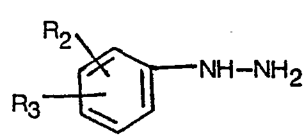

III

worin $R_2$ und $R_3$ die obige Formel haben, zu einer Verbindung der Formel

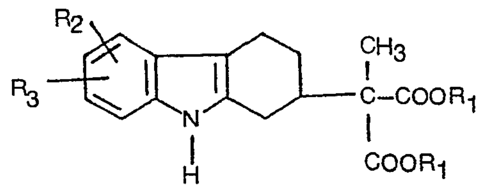

IV

worin $R_1$, $R_2$ und $R_3$ die obige Bedeutung haben, umsetzt,

b) die Verbindung der Formel IV mit einem Oxidationsmittel zu einer Verbindung der Formel

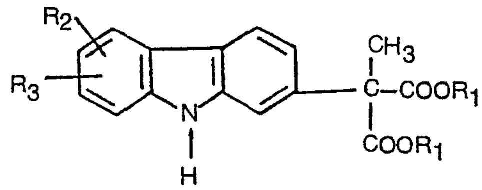

V

worin $R_1$, $R_2$ und $R_3$ die obige Bedeutung haben, umsetzt,

c) die Verbindung der Formel V hydrolysiert und decarboxyliert.

2. Ein Verfahren gemäss Anspruch 1, in dem p-Chlorphenylhydrazin als Ausgangsmaterial der Formel III verwendet wird.

3. Eine Verbindung der Formel

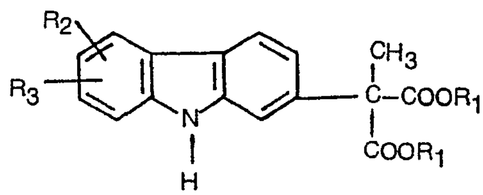

V

worin $R_1$ $C_{1-7}$-Alkyl, $R_2$ Wasserstoff, Halogen, Trifluormethyl, Hydroxy, $C_{1-7}$-Alkyl, Hydroxy-$C_{1-7}$-alkyl, $C_{1-7}$-Alkylthio, Amino, Mono-$C_{1-7}$-alkylamino oder Di-$C_{1-7}$-alkylamino; und $R_3$ Halogen, Trifluormethyl, $C_{1-7}$-Alkyl, Hydroxy-$C_{1-7}$-Alkyl, $C_{1-7}$-Alkoxy, $C_{1-7}$-Alkylthio, Hydroxy, Amino, Mono-$C_{1-7}$-alkylamino oder Di-$C_{1-7}$-alkylamino, oder $R_2$ zusammen mit einem benachbarten $R_3$ $C_{1-7}$-Alkylendioxy sind.

4. Diäthyl-(6-chlor-2-carbazolyl)-methylmalonat.

**Revendications**

1. Un procédé pour la préparation d'un composé de formule

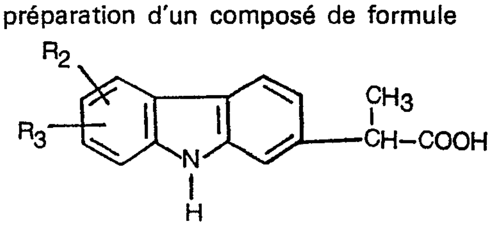

I

dans laquelle $R_2$ représente l'hydrogène, un halogène, un groupe trifluorométhyle, hydroxy, alkyle en C1—C7, hydroxyalkyle en C1—C7, alkylthio en C1—C7, amino, monoalkylamino en C1—C7 ou di-(alkyle en C1—C7)-amino; et $R_3$ représente un halogène, un groupe trifluorométhyle, alkyle en C1—C7, hydroxyalkyle en C1—C7, alcoxy en C1—C7, alkylthio en C1—C7, hydroxy, amino, monoalkylamino en C1—C7 ou di-(alkyle en C1—C7)-amino, ou bien $R_2$, pris avec un $R_3$ voisin, représente un groupe (alkylène en C1—C7)-dioxy, par utilisation en tant que produits de départ d'un dérivé de la cyclohexanone et d'une phénylhydrazine connue de formule III ci-après, ce procédé comprenant

a) la réaction d'un ester dialkylique inférieur de l'acide $\alpha$-méthyl-3-oxocyclohexane-malonique de formule

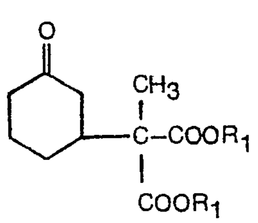

II

dans laquelle $R_1$ représente un groupe alkyle inférieur, avec une phénylhydrazine de formule

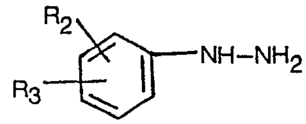

III

dans laquelle $R_2$ et $R_3$ ont les significations indiquées ci-dessus, qui donne un composé de formule

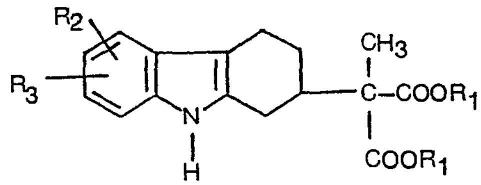

IV

dans laquelle $R_1$, $R_2$ et $R_3$ ont les significations indiquées ci-dessus,

b) le traitement du composé de formule IV par un agent oxydant, conduisant à un composé de formule

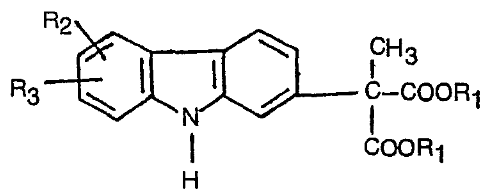

V

dans laquelle $R_1$, $R_2$ et $R_3$ ont les significations indiquées ci-dessus,

c) l'hydrolyse et la décarboxylation du composé de formule V.

2. Un procédé selon la revendication 1, dans lequel on utilise la p-chlorophénylhydrazine en tant que produit de départ de formule III.

3. Un composé de formule

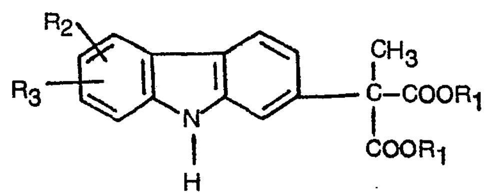

V

dans laquelle $R_1$ représente un groupe alkyle en C1—C7, $R_2$ représente l'hydrogène, un halogène, un groupe trifluorométhyle, hydroxy, alkyle en C1—C7, hydroxyalkyle en C1—C7, alkylthio en C1—C7, amino, monoalkylamino en C1—C7 ou di-(alkyle en C1—C7)-amino; et $R_3$ représente un halogène, un groupe trifluorométhyle, alkyle en C1—C7, hydroxyalkyle en C1—C7, alcoxy en C1—C7, alkylthio en C1—C7, hydroxy, amino, monoalkylamino en C1—C7 ou di-(alkyle en C1—C7)-amino ou bien $R_2$, pris avec un $R_3$ voisin, représente un groupe (alkylène en C1—C7)-dioxy.

4. Le (6-chloro-2-carbazolyl)-méthylmalonate de diéthyle.